# EUROPEAN PATENT SPECIFICATION

(11) **EP 3 410 956 B1**
(45) Date of publication and mention of the grant of the patent: **01.04.2020**
(21) Application number: 17707186.7
(22) Date of filing: 03.02.2017
(51) Int. Cl.: A61B 17/11

(54) **ANASTOMOTIC CONNECTOR**
ANASTOMOTISCHER VERBINDER
CONNECTEUR ANASTOMOTIQUE

(30) Priority: 05.02.2016 EP 16305143
(43) Date of publication of application: 12.12.2018
(73) Proprietor: CHU de Nice, 06000 Nice (FR)
(72) Inventor: ROSELLO, Olivier, 06000 Nice (FR)
(74) Representative: Osha Liang
(86) International application number: PCT/EP2017/052456
(87) International publication number: WO 2017/134267

(56) References cited:
- EP-A1- 1 908 420
- WO-A1-99/55254
- FR-A1- 2 683 141
- US-A- 4 214 586
- US-A1- 2001 039 425

## Description

### TECHNICAL FIELD

The present disclosure relates generally to an anastomotic connector, that is a device for interconnecting separate or severed tubular hollow structures to form a continuous channel. In particular, it relates to a vascular anastomotic connector.

### BACKGROUND

A vascular anastomosis is a connection between two blood vessels. The connection may be established between two vessel ends (then, it is referred to an end-to-end anastomosis), or between the end of one vessel and the side of another vessel (then, it is referred to an end-to-side anastomosis). The vessels may be natural or prosthetic.

Vascular anastomoses are still today widely carried out by suturing the vessels with a non-resorbable surgical thread or by using surgical staples. Although globally satisfactory, these techniques take time and, if not performed correctly, may cause blood loss or stenosis in the vessel-connection area.

Suturing means for connecting a tubular vascular prosthesis to a blood vessel are disclosed in patent document WO 99/55254. As an alternative to sutures and surgical staples, anastomotic connectors may be used.

Examples of anastomotic connectors are described, for instance, in patent document FR 2683141. These connectors have two opposite ends to be inserted, respectively, into vessel ends. The connector end may have barbs or circumferential ridges on its external surface, for preventing the vessel from sliding out of the connector end. An external clamp is provided around the vessel portion overlying the connector end to hold the vessel in place on the connector.

While conventional anastomotic connectors have proven effective, because of the constant micro-motions of the vessels (due to the oscillating blood pressure), blood leakage may happen in the vessel-connection area and the vessel may slide out of the connector end, even when an external clamp is provided. Further, if the external clamp is clamped too tight around the vessel, there is a risk of damaging the vessel portion overlying the connector and, in particular, of cutting circumferentially the vessel. If the vessel is damaged by the external clamp, this may, in turn, be detrimental to the leak-tightness of the connection or to the fixing of the vessel.

Accordingly, there exists a need for a new kind of anastomotic connector addressing the aforementioned problems, or at least providing the practitioners with a useful alternative to the conventional connectors.

### GENERAL PRESENTATION

An anastomotic connector according to the invention is defined in the appended claims. Such an anastomotic connector comprises: a connector body having a first end engageable into a first vessel, and a first locking sleeve engageable around the first end. The first end of the connector body is provided with teeth on its outer surface, and the first locking sleeve surrounds the teeth in a locked position, in order to impede relative movement between the first vessel and the connector body. The first locking sleeve is provided with longitudinal ribs on its inner surface. The ribs are spaced from each other in a circumferential direction of the sleeve.

When the first locking sleeve surrounds the teeth, the first vessel is trapped between the first locking sleeve and the connector body, the ribs pushing the vessel towards the teeth. Thus, the risk that the vessel disengages from the teeth is limited and the leak-tightness between the connector body and the vessel is improved.

Moreover, the risk of damaging the first vessel is limited due to the orientation and position of the ribs. In particular, as compared to a circumferential ridge or analog, the risk of cutting or constricting circumferentially the first vessel is reduced. This is beneficial for the fixing of the first vessel and for the growth of tissue cells around this vessel.

The first locking sleeve has a main axis, or central line, extending between its two main opposite openings and passing through the center of each cross-section of the sleeve. In the frame of the present disclosure, the length of the sleeve is the dimension along the main axis of the sleeve. The ribs are said to be longitudinal because they extend substantially in a longitudinal direction of the sleeve. A circumferential direction follows the circumference of a cross-section of the sleeve. A radial direction is a direction perpendicular to the main axis. The "inner" and "outer" parts or surfaces of an element are considered in relation to the radial direction. Thus, for instance, in case of a sleeve having a cylindrical shape, the main axis is the revolution axis. A longitudinal direction is parallel to the main axis, a circumferential direction is circular around the main axis and a radial direction is perpendicular to the main axis. The same applies for the connector body. Finally, the words "distal" and "proximal" are considered in relation to the center of the connector and lengthwise; one element (or one part of element) is proximal to another element (or another part of the element) when it is closer to the center of the connector than the other element (or the other part of the element), lengthwise.

The teeth provided on the outer surface of the connector end may have various shapes: they may have various thicknesses, various heights, be more or less sharp, etc. For instance, the teeth may be barbs, spikes, small peaks, etc. Also, the teeth may be in various positions. In particular, they may be arranged on the outer surface in at least two circumferential rows spaced from each other. This allows the vessel to be better held on the connector end. The teeth may be all the same or different from each other, depending on their position.

In certain embodiments, there are two rows of teeth spaced from each other by a first distance, while the longitudinal ribs have a length greater than or equal to the first distance and extend over the rows of teeth in the locked position. This allows the vessel to be better held on the connector end.

In certain embodiments, the first locking sleeve is provided with through holes or slots extending from the inner surface to the outer surface of the locking sleeve. Thus, after implantation, tissue cells may grow inside the holes or slots, from the vessel to the outside of the locking sleeve.

In certain embodiments, the first locking sleeve and the first end are provided with a locking system that maintains the first locking sleeve in a fixed position relative to the first end, the fixed position corresponding to said locked position. The locking system may be such that, for moving the first locking sleeve to the locked position, the first locking sleeve has first to be moved lengthwise onto to the first end and then rotated around the first end. When the locking sleeve is turned, the ribs may pass over the teeth and push the vessel onto the teeth, thereby helping the teeth to penetrate into the vessel thickness. The rotation of the sleeve may, however, be limited to avoid damaging the vessel. For instance, the rotation may not exceed a quarter or one-eighth of a turn.

The connector body and the first locking sleeve may be spaced from each other by a radial gap when the first locking sleeve is in the locked position. The height of the teeth and/or the height of the ribs may be both lower than the radial gap. If not, the teeth and/or the ribs are deformable. In addition, the sum of the tooth height and the rib height may be lower than the radial gap. If not, the ribs are sufficiently deformable to pass over the teeth, or the teeth are sufficiently deformable to pass under the ribs, when the first locking sleeve is rotated around the first end.

When the first locking sleeve is in the locked position, the ribs are fitted in between the teeth. In particular, this means that the ribs are fitted in between the teeth after the first locking sleeve has been rotated to its locked position. Thus, the pressure applied on the vessel is limited (as compared to a situation where the ribs are in front of the teeth in the locked position), which decreases the risk of damaging the vessel in the long term.

In certain embodiments, the connector body of the anastomotic connector has a second end similar to the first end and opposed thereto, the second end being engageable into a second vessel. Further, the connector is provided with a second locking sleeve, similar to the first locking sleeve, engageable around the second end in order to impede relative movement between the second vessel and the connector body.

In other embodiments, the anastomotic connector comprises another similar connector body, i.e. it comprises two connector bodies similar to each other. The anastomotic connector further comprises a second locking sleeve, similar to the first locking sleeve, and engageable around the first end of the other connector body. Each connector body has a second end opposed to the first end, and the second ends of the connector bodies are connectable to each other. In other words, the two connector bodies may be connected together through their second ends. In particular, the second ends of the connectors may have complementary shapes, fitting into each other. In such a case, the two connector bodies may be identical to each other, apart from their second ends.

According to another aspect of the present disclosure, there is provided a kit comprising an anastomotic connector as described above and an insertion sleeve engageable around the first end of the connector body for covering the teeth, the insertion sleeve being removable from the connector body. The insertion sleeve is used for making the insertion of the connector body into the vessel easier. Then, once the connector body is in proper position inside the vessel, the insertion sleeve is removed, thus uncovering the teeth of the connector body, and the teeth penetrate into the vessel wall.

In certain embodiments, the insertion sleeve is tapered from a first end with a wide opening to a second end with a narrow opening. The connector body is inserted into the insertion sleeve through the wide opening, and the insertion sleeve is deformable so that the connector body can deform the narrow opening and pass therethrough. Thus, for removing the insertion sleeve, it is possible to pull the first end of the sleeve, while maintaining the connector body in place, so that the second end of the sleeve passes over the connector body.

In certain embodiments, the section of the narrow opening is smaller than the smallest cross-section of the connector body, when the insertion sleeve is not deformed, and preferably smaller than the vessel cross-section. The second end of the insertion sleeve can thus be inserted into the vessel more easily than the first end of the connector body.

In certain embodiments, the kit further comprises a push handle engageable within the connector body for pushing the connector body into the first vessel and/or the insertion sleeve.

According to another aspect of the present disclosure, there is provided a method for anastomosis comprising the steps of: providing an anastomotic connector as described above, engaging the first end of the connector body into the first vessel, and moving the first locking sleeve to its locked position in order to impede relative movement between the connector and the first vessel. Such a method may be used for establishing a connection between two vessel ends, i.e. for an end-to-end anastomosis, or for establishing a connection between the end of one vessel and the side of another vessel, i.e. for an end-to-side anastomosis. A connector body with two similar opposite ends provided with teeth may be used for performing an end-to-end anastomosis and the method may further comprise: engaging the second end of the connector body into the second vessel, and moving the second locking sleeve to its locked position in order to impede relative movement between the connector and the second vessel.

Two connector bodies connectable with each other may also be used for performing an end-to-end anastomosis and the method may further comprise: engaging the first end of the second connector body into the second vessel, moving the second locking sleeve to its locked position in order to impede relative movement between the second connector body and the second vessel, and connecting together the second ends of the connector bodies.

Before engaging the first end of the connector body into the first vessel, the first locking sleeve may be slipped on the first vessel. Similarly, before engaging the second end of the connector body into the second vessel, the second locking sleeve may be slipped on the second vessel.

Such methods have the advantages derived from using an anastomotic connector according to the present disclosure.

Other features and advantages of the invention will become apparent from the following detailed description, taken in conjunction with the accompanying drawings, which illustrate, by way of example, the principles of the invention.

### BRIEF DESCRIPTION OF THE DRAWINGS

In the drawings, like reference signs generally refer to the same or like parts throughout the different views. The drawings are not necessarily to scale, emphasis instead being placed upon illustrating the principles of the invention.
FIG 1 is a perspective view of an example of an anastomotic connector including a connector body and two locking sleeves, respectively engaged around the two opposite ends of the connector body.
FIG 2 is a side view of the connector body of FIG 1, following arrow II.
FIG 3 is a front view of the connector body of FIG 1, following arrow III.
FIG 4 is a perspective front view of one of the locking sleeves of FIG 1.
FIG 5 is a perspective back view of one of the locking sleeves of FIG 1.
FIGS 6 and 7 are side views of another example of anastomotic connector including two connector bodies and two locking sleeves. In FIG 6, the connector bodies are not connected together, while they are in FIG 7.
FIGS 8 to 11 illustrate a kit comprising an anastomotic connector, an insertion sleeve and a push handle, and the way the kit is used.

### DETAILED DESCRIPTION

In the following detailed description, it is referred to the accompanying drawings showing an example of anastomotic connector. It is intended that this example be considered as illustrative only, the invention not being limited to this example.

To avoid unnecessary details for practicing the invention, the description may omit certain information already known to those skilled in the art.

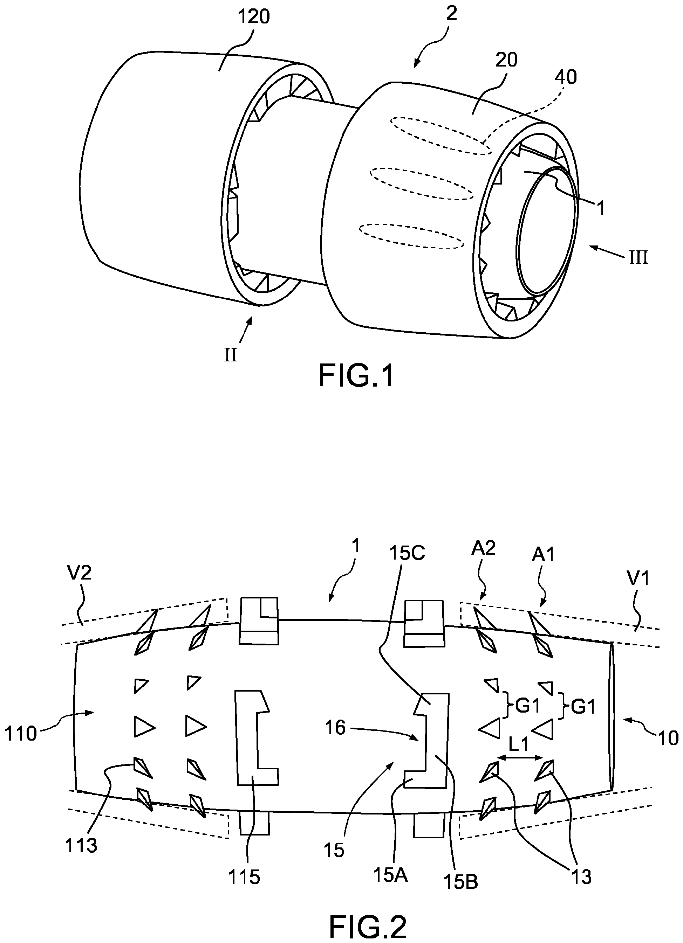

With reference to the figures, the reference number 2 generally designates an anastomotic connector, that is a connector for establishing a connection between two blood vessels V1, V2, shown in dotted line in FIG 2. In this example, the connector 2 is an end-to-end anastomotic connector, which means that it establishes a connection between a free end of a first vessel V1 and a free end of a second vessel V2. The connector 2 comprises a connector body 1 with two opposite ends 10 and 110. The first and second ends 10, 110 are adapted for being engaged into the first and second vessels V1, V2, respectively. To make this engagement easier, the first and second ends 10, 110 may be tapered, as illustrated in FIG 2. The first and second ends 10, 110 are provided with teeth 13, 113 on their outer surface. These teeth 13 are adapted to penetrate into the vessel inner wall for holding the vessels V1, V2 in position on the first and second ends 10, 110.

The connector 2 further comprises two locking sleeves 20, 120 which are adapted for being engaged around the first and second ends 10, 110, respectively. In FIG 1, the first and second locking sleeves 20, 120 are shown in their locked position. In this locked position, the locking sleeves 20, 120 overly the teeth 13, 113, respectively.

In the illustrated example, the two opposite ends of the connector 2 are similar. In particular, the first and second ends 10, 110 of the connector body 1 are similar, and the locking sleeves 20, 120 are similar. Therefore, for the sake of conciseness, only the first connector end 10 with the locking sleeve 20 will be described below.

In order to maintain the first locking sleeve 20 in its locked position on the first end 10, the locking sleeve 20 and the first end 10 are provided with hooking parts 15, 25 cooperating with each other. In the illustrated example, the hooking parts 15 protrude on the outer surface of the first end 10 and are proximal to the teeth 13 (i.e. closer to the center of the connector body 1 than the teeth 13, lenghtwise). The hooking parts 15 are circumferentially spaced from each other. At least one hooking part 15 (each hooking part 15 in the example) comprises a base 15B and two branches 15A, 15C extending from the base 15B in the proximal direction, thereby forming a "U" with a notch 16 defined between the base 15B and the branches 15A, 15C. The notch 16 is on the proximal side of the hooking part 15. The hooking parts 25 of the locking sleeve 20 are tabs protruding from the inner surface and located at the proximal end of sleeve 20. The hooking parts 25 are circumferentially spaced from each other and adapted to fit in the notches 16. For moving the hooking parts 25 into the notches 16, the first locking sleeve 20 has first to be slipped onto to the first end 10, so that each hooking part 25 passes between and goes beyond two adjacent hooking parts 15. Then the locking sleeve 20 is rotated around the first end 10, so that the hooking part 25 passes over the branch 15C. To facilitate this step, the end of the branch 15C may be sloped, as illustrated in FIG 2. Then, the hooking part 25 engages into the notches 16 and the locking sleeve 20 is released. When the hooking parts 25 are engaged in the notches 16, the first locking sleeve 20 is held in a fixed position relative to the first end 10, corresponding to the locked position.

Going back to the teeth 13, they are arranged on the outer surface in at least two circumferential rows A1, A2 spaced from each other, lengthwise, by a first distance L1. In each row A1, A2, the teeth 13 are separated from each other by circumferential gaps G1. Also, the teeth 13 of the rows A1, A2 are aligned in a longitudinal direction of the connector body 2, so that gaps G1 are aligned in a longitudinal direction.

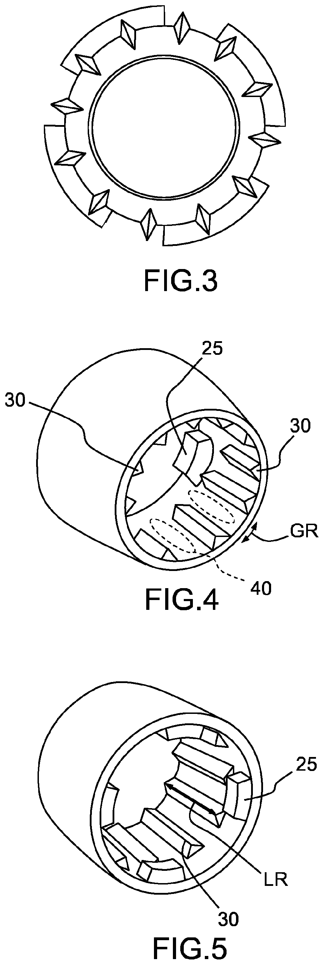

As shown in FIGS 4 and 5, the sleeve 20 is provided with longitudinal ribs 30 on its inner surface. The ribs 30 have a length LR greater than or equal to the first distance L1, and are spaced from each other in a circumferential direction of the first locking sleeve by a circumferential gap GR. When the first locking sleeve is held in the fixed position, each rib 30 extends longitudinally over the tooth rows A1, A2, while being fitted circumferentially in between adjacent teeth 13. The ribs 30 may be tapered towards their ridge. In particular, the ribs 30 may have a thin ridge line and their cross-section is substantially triangular.

The height of the ribs 30 may be such that, while the ribs 30 are fitted circumferentially in between adjacent teeth 13, the ridge of a rib 30 extends radially beyond the tips of the adjacent teeth 13, i.e. the ridge of the rib 30 is closer to the connector body 1 than the tips of the adjacent teeth 13.

Finally, the sleeve 20 may be provided with through holes or slots 40 (shown in dotted lines in FIGS 1 and 4) extending from the inner surface to the outer surface of the sleeve, for promoting tissue growth through the sleeve 20 after implantation. The slots 40 may extend longitudinally between the longitudinal ribs 30.

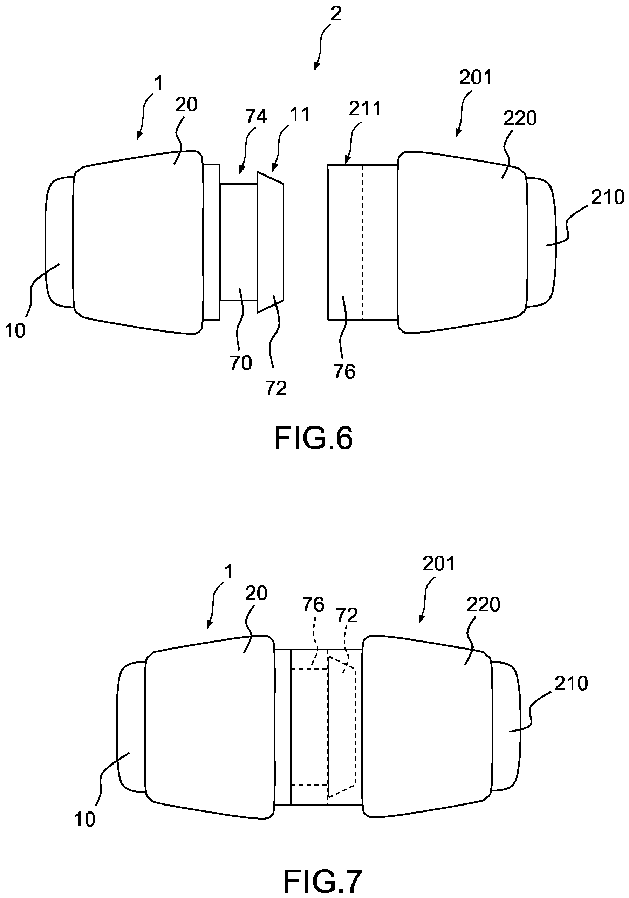

FIGS 6 and 7 illustrate another example of an anastomotic connector 2 for establishing an end-to-end connection between two blood vessels, i.e. for performing an end-to-end anastomosis. The connector 2 comprises two connector bodies 1, 201 and two locking sleeves 20, 220. The first ends 10, 210 of the connector bodies are adapted for being engaged into first and second vessels (not shown), respectively, and both comprise teeth (not shown) on their outer surface. The connector 2 further comprises two locking sleeves 20, 220 with longitudinal ribs (not shown) which are adapted for being engaged around the first ends 10, 210, respectively. In the figures, the first and second locking sleeves 20, 220 are shown in their locked position, where they overly the teeth of the first ends 10, 210, respectively. The first ends 10, 210 and the first and second locking sleeves 20, 220 are similar, respectively, to the first and second ends 10, 110 and the first and second locking sleeves 20, 120 shown in FIGS 1-5. Therefore, for the sake of conciseness, they will not be described again.

The connector bodies 1, 201 have second ends 11, 211, opposite to their first ends 10, 210. The second ends 11, 211 can be mechanically connected together.

In the illustrated example, the second end 11 is provided with a longitudinal tubular extension 70. The outer surface of the tubular extension 70 is provided with a circumferential groove 74 followed by an external circumferential rib 72 at the free end of the tubular extension 70. The second end 211 is provided, on its inner surface with an internal circumferential rib 76. The bodies 1, 201 are connected together by inserting the tubular extension 70 in force into the second end 211. In order to guide and make the insertion easier, the circumferential rib 72 is truncated. During the insertion, the external circumferential rib 72 goes beyond the internal circumferential rib 76 and the rib 76 fits into the circumferential groove 74, so that the tubular extension 70 gets locked into the second end 211, as shown in FIG. 7. Of course, other kinds of connection system could be used for connecting the second ends 11, 211 together, such as systems with hooks, elastic tabs, small successive circumferential ribs, etc.

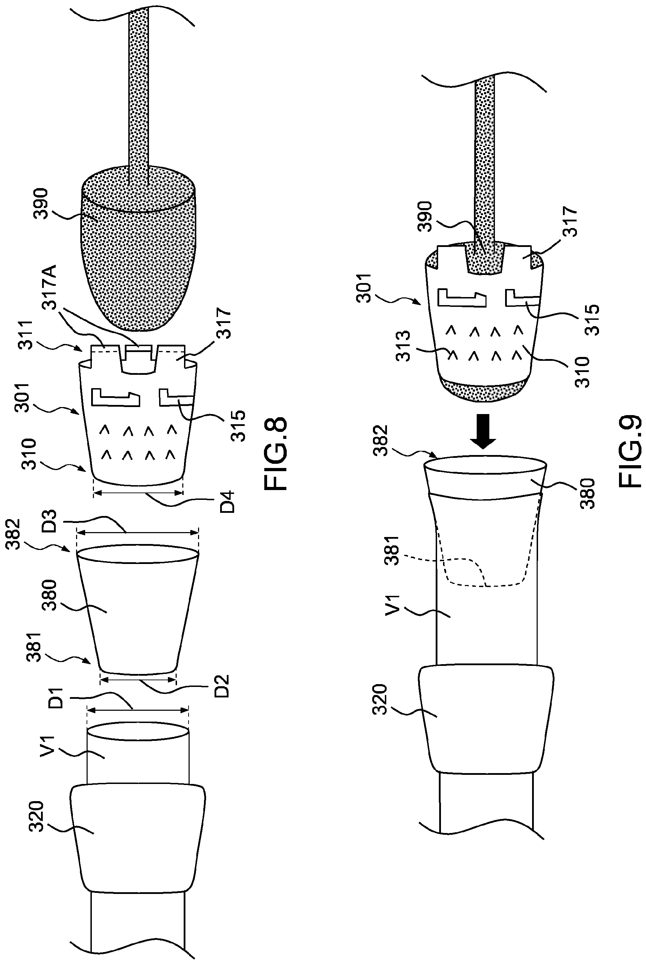

FIGS 8-11 illustrate, diagrammatically, a kit comprising another example of an anastomotic connector, an insertion sleeve 380 and a push handle 390, and the way the kit is used.

Like the connector 2 of FIGS 6-7, the connector of FIGS 8-11 comprises first and second connector bodies connectable together, but only the first connector body 301 is shown in the drawings. The first end 310 of the connector body 301 is adapted for being engaged into a first vessel V1. The outer surface of the first end 310 is provided with teeth 313 adapted to penetrate into the inner wall of the vessel V1, for holding the vessel V1 in position on the first end 310. The outer face is also provided with hooking parts 15 cooperating with other hooking parts (not shown) of the locking sleeve 320. The second end 311 of the connector body 301 is provided with a connection system for connecting the first connector body 301 to a second connector body (not shown). The connection system comprises elastic tabs 317 provided with a hook 317A at their distal ends, the hook being engageable with a corresponding slot (not shown) provided on the second end of the second connector body (not shown).

The insertion sleeve 380 is tapered from a first end 382 with a wide opening to a second end 381 with a narrow opening. In the example, these openings have a circular section of diameters D2 and D3, with D2<D3. The section of the narrow opening is smaller than the smallest cross-section of the connector body 301, when the insertion sleeve is not deformed. In other words, in the example, the diameter D2 is smaller than the diameter D4 of the tip of the connector first end 310. The diameter D2 is also smaller than the vessel diameter D1. Thus, the first end 381 of the insertion sleeve 380 can be inserted more easily into the vessel V1 than the first end 310 of the connector body 301, and the first end 310 of the connector body 301 can be inserted easily into the insertion sleeve 380, as illustrated by the black arrow in FIG 9. The push handle 390 is used for holding and pushing the connector body 301 into the insertion sleeve 380.

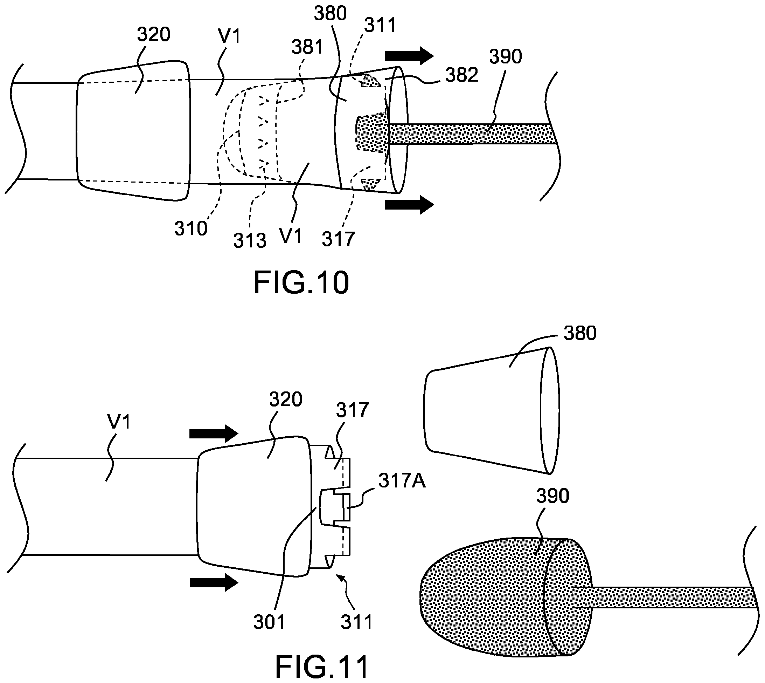

Once the connector body 301 is in proper position inside the vessel V1, the insertion sleeve 380 is removed, thus uncovering the teeth 313 of the connector body, as illustrated in FIG 10. The teeth 313 can then penetrate into the vessel wall. For removing the insertion sleeve 380, the first end 382 of the sleeve is pulled out, as illustrated by the black arrows in FIG 10, while maintaining the connector body 301 in place by means of the push handle 390. Since the insertion sleeve 380 is deformable, the second end 381 of the sleeve 380 passes over the connector body 301 and the insertion sleeve 380 is removed.

Once the insertion sleeve 380 is removed, the locking sleeve 320 is engaged onto the teeth 313 of the connector body 310, as illustrated by the black arrows in FIG 11.

While only few embodiments have been chosen to illustrate the present invention, it will be apparent to those skilled in the art from this disclosure that various changes and modifications can be made herein without departing from the scope of the invention as defined in the appended claims. For example, the size, shape, location or orientation of the various components can be changed as needed and/or desired. Further, it is not necessary for all advantages to be present in a particular embodiment at the same time.

## Claims

1. An anastomotic connector comprising:
a connector body (1) having a first end (10) engageable into a first vessel (V1), the first end being provided with teeth (13) on its outer surface,
a first locking sleeve (20) engageable around the first end (10), the first locking sleeve (20) surrounding the teeth (13) in a locked position, in order to impede relative movement between the first vessel (V1) and the connector body (1),
wherein the first locking sleeve (20) is provided with longitudinal ribs (30) on its inner surface, the ribs being spaced from each other in a circumferential direction of the first locking sleeve (20), and **characterised in that** the ribs (30) are configured to be fitted in between the teeth (13) when the first locking sleeve (20) is in the locked position.

2. The anastomotic connector of claim 1, wherein the teeth (13) are arranged on the outer surface in at least two circumferential rows (A1, A2) spaced from each other.

3. The anastomotic connector of claim 2, wherein the two rows of teeth (13) are spaced from each other by a first distance (D1), wherein the ribs (30) have a length (LR) greater than or equal to the first distance (D1), and wherein the ribs (30) extends over the rows of teeth (A1, A2) in the locked position.

4. The anastomotic connector of any of claims 1 to 3, wherein the first locking sleeve (20) is provided with through holes or slots (40) extending from the inner surface to the outer surface of the first locking sleeve (20).

5. The anastomotic connector of any of claims 1 to 4, wherein the first locking sleeve (20) and the first end (10) are provided with a locking system that maintains the first locking sleeve (20) in a fixed position relative to the first end (10), corresponding to said locked position.

6. The anastomotic connector of claim 5, wherein the locking system is such that, for moving the first locking sleeve (20) to the locked position, the first locking sleeve (20) has first to be moved lengthwise onto to the first end (10) and then rotated around the first end (10).

7. The anastomotic connector of any of claims 1 to 6, wherein the ribs (30) are sufficiently deformable to pass over the teeth (13) when the first locking sleeve (20) is rotated around the first end (10).

8. The anastomotic connector of any of claims 1 to 7, wherein the locking system comprises hooking parts (15, 25) provided on both the first end (10) and the first locking sleeve (20), and cooperating with each other for maintaining the first locking sleeve (20) in the locked position.

9. The anastomotic connector of any of claims 1 to 8, wherein , when the ribs (30) are fitted in between the teeth (13) when the first locking sleeve (20) is in the locked position, the ridge of each rib (30) is closer to the connector body (1) than the tips of the adjacent teeth (13).

10. The anastomotic connector of any of claims 1 to 9, wherein the connector body (1) has a second end (110) provided with teeth (113) on its outer surface, like the first end (10), the second end (110) being opposed to the first end (10) and engageable into a second vessel (V2), the connector comprising a second locking sleeve (120) provided with longitudinal ribs on its inner surface, like the first locking sleeve (20), and being engageable around the second end (110) in order to impede relative movement between the second vessel (V2) and the connector body (1).

11. The anastomotic connector of any of claims 1 to 8, comprising :
a second connector body (201) having a first end (210) provided with teeth on its outer surface, like the first end (10) of the first connector body (1), and
a second locking sleeve (220) provided with longitudinal ribs on its inner surface, like the first locking sleeve (20), and engageable around the first end (210) of the second connector body (201),
wherein each connector body (1, 201) has a second end (11, 211) opposed to the first end (10, 210), and the second ends (11, 211) of the connector bodies are connectable to each other.

12. A kit comprising an anastomotic connector according to any one of the preceding claims and an insertion sleeve (380) engageable around the first end (301) of the connector body (301) for covering the teeth (313), the insertion sleeve (380) being removable from the connector body (301).

13. A kit according to claim 12, wherein the insertion sleeve (380) is tapered from a first end (382) with a wide opening to a second end (381) with a narrow opening, wherein the connector body (301) can be inserted into the insertion sleeve (380) through the wide opening, and wherein the insertion sleeve (380) is deformable so that the connector body (301) can deform the narrow opening and pass therethrough.

14. A kit according to claim 13, wherein the section of the narrow opening is smaller than the smallest cross-section of the connector body (301), when the insertion sleeve (380) is not deformed.

15. A kit according to any one of claims 12 to 14, further comprising a push handle (390) engageable within the connector body (301) for pushing the connector body (1) into the first vessel (V1) and/or the insertion sleeve (380).

## Patentansprüche

1. Anastomotischer Verbinder, der aufweist:
einen Verbinderkörper (1) mit einem ersten Ende (10), das in ein erstes Gefäß (V1) eingreift, wobei das erste Ende mit Zähnen (13) auf seiner Außenfläche versehen ist,
eine erste Verrastungshülse (20), die um das erste Ende (10) herum angreift, wobei die erste Verrastungshülse (20) in einer Verrastungsposition die Zähne (13) umgibt, um eine Relativbewegung zwischen dem ersten Gefäß (V1) und dem Verbinderkörper (1) zu verhindern,
wobei die erste Verrastungshülse (20) mit Längsrippen (30) auf ihrer Innenfläche versehen ist, wobei die Rippen in Umfangsrichtung der ersten Verrastungshülse (20) voneinander beabstandet sind, und
**dadurch gekennzeichnet, dass**
die Rippen (30) derart ausgebildet sind, dass sie zwischen den Zähnen (13) eingepasst sind, wenn die erste Verrastungshülse (20) in der Verrastungsposition ist.

2. Anastomotischer Verbinder nach Anspruch 1, bei dem die Zähne (13) voneinander beabstandet in mindestens zwei Umfangsreihen (A1, A2) auf der Außenfläche angeordnet sind.

3. Anastomotischer Verbinder nach Anspruch 2, bei dem die zwei Reihen von Zähnen (13) um einen ersten Abstand (D1) voneinander beabstandet sind, wobei die Rippen (30) eine Länge (LR) haben, die größer als der oder gleich dem ersten Abstand (D1) ist, und wobei sich die Rippen (30) in der Verrastungsposition über die Reihen von Zähnen (A1, A2) erstrecken.

4. Anastomotischer Verbinder nach einem der Ansprüche 1 bis 3, bei dem die erste Verrastungshülse (20) mit Durchgangslöchern oder Schlitzen (40) versehen ist, die sich von der Innenfläche zu der Außenfläche der ersten Verrastungshülse (20) erstrecken.

5. Anastomotischer Verbinder nach einem der Ansprüche 1 bis 4, bei dem die erste Verrastungshülse (20) und das erste Ende (10) mit einem Verrastungssystem versehen sind, das die erste Verrastungshülse (20) relativ zu dem ersten Ende (10) in einer festen Position, die der Verrastungsposition entspricht, hält.

6. Anastomotischer Verbinder nach Anspruch 5, bei dem das Verrastungssystem ein solches ist, dass zum Bewegen der ersten Verrastungshülse (20) in die Verrastungsposition die erste Verrastungshülse (20) zuerst in Längsrichtung auf das erste Ende (10) bewegt und dann um das erste Ende (10) herum gedreht werden muss.

7. Anastomotischer Verbinder nach einem der Ansprüche 1 bis 6, bei dem die Rippen (30) ausreichend verformbar sind, um sich über die Zähne (13) zu bewegen, wenn die erste Verrastungshülse (20) um das erste Ende (10) herum gedreht wird.

8. Anastomotischer Verbinder nach einem der Ansprüche 1 bis 7, bei dem das Verrastungssystem Hakenteile (15, 25) aufweist, die sowohl am ersten Ende (10) als auch an der ersten Verrastungshülse (20) vorgesehen sind und zusammenwirken, um die erste Verrastungshülse (20) in der Verrastungssposition zu halten.

9. Anastomotischer Verbinder nach einem der Ansprüche 1 bis 8, bei dem dann, wenn die Rippen (30) zwischen den Zähnen (13) eingepasst sind, wenn die erste Verrastungshülse (20) in der Verrastungsposition ist, der Grat jeder Rippe (30) näher an dem Verbinderkörper (1) ist als die Spitzen der angrenzenden Zähne (13).

10. Anastomotischer Verbinder nach einem der Ansprüche 1 bis 9, bei dem der Verbinderkörper (1) ein zweites Ende (110) aufweist, das wie das erste Ende (10) mit Zähnen (113) auf seiner Außenfläche versehen ist, wobei das zweite Ende (110) dem ersten Ende (10) gegenüberliegt und in ein zweites Gefäß (V2) eingreift, wobei der Verbinder eine zweite Verrastungshülse (120) aufweist, die wie die erste Verrastungshülse (20) mit Längsrippen auf ihrer Innenfläche versehen ist und um das zweite Ende (110) herum angreift, um eine Relativbewegung zwischen dem zweiten Gefäß (V2) und dem Verbinderkörper (1) zu verhindern.

11. Anastomotischer Verbinder nach einem der Ansprüche 1 bis 8, der aufweist:
einen zweiten Verbinderkörper (201) mit einem ersten Ende (210), das wie das erste Ende (10) des ersten Verbinderkörpers (1) mit Zähnen auf seiner Außenfläche versehen ist, und
eine zweite Verrastungshülse (220), die wie die erste Verrastungshülse (20) mit Längsrippen auf ihrer Innenfläche versehen ist und um das erste Ende (210) des zweiten Verbinderkörpers (201) herum angreift,
wobei jeder Verbinderkörper (1, 201) ein zweites Ende (11, 211) gegenüber dem ersten Ende (10, 210) aufweist und die zweiten Enden (11, 211) der Verbinderkörper miteinander verbindbar sind.

12. Kit, das einen anastomotischen Verbinder nach einem der vorhergehenden Ansprüche und eine Einsetzhülse (380) aufweist, die zum Abdecken der Zähne (313) um das erste Ende (301) des Verbinderkörpers (301) herum angreift, wobei die Einsetzhülse (380) von dem Verbinderkörper (301) lösbar ist.

13. Kit nach Anspruch 12, bei dem sich die Einsetzhülse (380) von einem ersten Ende (382) mit einer breiten Öffnung zu einem zweiten Ende (381) mit einer engen Öffnung verjüngt, wobei der Verbinderkörper (301) durch die breite Öffnung in die Einsetzhülse (380) eingesetzt werden kann und wobei die Einsetzhülse (380) derart verformbar ist, dass der Verbinderkörper (301) die enge Öffnung verformen und sich durch diese hindurch bewegen kann.

14. Kit nach Anspruch 13, bei dem der Querschnitt der engen Öffnung kleiner ist als der kleinste Querschnitt des Verbinderkörpers (301), wenn die Einsetzhülse (380) nicht verformt ist.

15. Kit nach einem der Ansprüche 12 bis 14, das ferner einen Schiebegriff (390) aufweist, der zum Drücken des Verbinderkörpers (1) in das erste Gefäß (V1) und/oder die Einsetzhülse (380) in den Verbinderkörper (301) eingreift.

## Revendications

1. Connecteur anastomotique comprenant :
un corps de connecteur (1) ayant une première extrémité (10) pouvant être engagée dans un premier vaisseau (V1), la première extrémité étant pourvue de dents (13) sur sa surface extérieure,
un premier manchon de verrouillage (20) pouvant être engagé autour de la première extrémité (10), le premier manchon de verrouillage (20) entourant les dents (13) dans une position verrouillée, afin d'empêcher un mouvement relatif entre le premier vaisseau (V1) et le corps de connecteur (1),
dans lequel le premier manchon de verrouillage (20) est pourvu des nervures longitudinales (30) sur sa surface intérieure, les nervures étant espacées les unes des autres dans une direction circonférentielle du premier manchon de verrouillage (20), et
**caractérisé en ce que** les nervures (30) sont configurées pour être insérées entre les dents (13) lorsque le premier manchon de verrouillage (20) est dans la position verrouillée.

2. Connecteur anastomotique selon la revendication 1, dans lequel les dents (13) sont agencées sur la surface extérieure en au moins deux rangées circonférentielles (A1, A2) espacées l'une de l'autre.

3. Connecteur anastomotique (1) selon la revendication 2, dans lequel les deux rangées de dents (13) sont espacées l'une de l'autre d'une première distance (D1), dans lequel les nervures (30) ont une longueur (LR) supérieure ou égale à la première distance (D1), et dans lequel les nervures (30) s'étendent par dessus les rangées de dents (A1, A2) dans la position verrouillée.

4. Connecteur anastomotique selon l'une quelconque des revendications 1 à 3, dans lequel le premier manchon de verrouillage (20) est pourvu de trous traversants ou de fentes (40) qui s'étendent à partir de la surface intérieure jusqu'à la surface extérieure du premier manchon de verrouillage (20).

5. Connecteur anastomotique selon l'une quelconque des revendications 1 à 4, dans lequel le premier manchon de verrouillage (20) et la première extrémité (10) sont pourvus d'un système de verrouillage qui maintient le premier manchon de verrouillage (20) dans une position fixe par rapport à la première extrémité (10), qui correspond à ladite position verrouillée.

6. Connecteur anastomotique selon la revendication 5, dans lequel le système de verrouillage est tel que, pour déplacer le premier manchon de verrouillage (20) dans la position verrouillée, le premier manchon de verrouillage (20) doit d'abord être déplacé dans le sens de la longueur sur la première extrémité (10) puis être tourné autour de la première extrémité (10).

7. Connecteur anastomotique selon l'une quelconque des revendications 1 à 6, dans lequel les nervures (30) sont suffisamment déformables pour passer par-dessus les dents (13) lorsqu'on fait tourner le premier manchon de verrouillage (20) autour de la première extrémité (10).

8. Connecteur anastomotique selon l'une quelconque des revendications 1 à 7, dans lequel le système de verrouillage comprend des parties d'accroche (15, 25) présentes, à la fois sur la première extrémité (10) et sur le premier manchon de verrouillage (20), et coopérant l'une avec l'autre pour maintenir le premier manchon de verrouillage (20) dans la position verrouillée.

9. Connecteur anastomotique selon l'une quelconque des revendications 1 à 8, dans lequel, lorsque les nervures (30) sont placées entre les dents (13), lorsque le premier manchon de verrouillage (20) est dans la position verrouillée, le faîte de chaque nervure (30) est plus proche du corps de connecteur (1) que les pointes des dents (13) adjacentes.

10. Connecteur anastomotique selon l'une quelconque des revendications 1 à 9, dans lequel le corps de connecteur (1) est pourvu d'une seconde extrémité (110) pourvue de dents (113) sur sa surface extérieure, comme la première extrémité (10), la seconde extrémité (110) étant opposée à la première extrémité (10) et pouvant être engagée dans un second vaisseau (V2), le connecteur comprenant un second manchon de verrouillage (120) pourvu de nervures longitudinales sur sa surface intérieure, comme le premier manchon de verrouillage (20), et pouvant être engagé autour de la seconde extrémité (110) afin d'empêcher un mouvement relatif entre le second vaisseau (V2) et le corps de connecteur (1).

11. Connecteur anastomotique selon l'une quelconque des revendications 1 à 8, comprenant :
un second corps de connecteur (201) qui présente une première extrémité (210) pourvue de dents sur sa surface extérieure, comme la première extrémité (10) du premier corps de connecteur (1), et
un second manchon de verrouillage (220) pourvu de nervures longitudinales sur sa surface intérieure, comme le premier manchon de verrouillage (20), et pouvant être engagé autour de la première extrémité (210) du second corps de connecteur (201),
dans lequel chaque corps de connecteur (1, 201) présente une seconde extrémité (11, 211) opposée à la première extrémité (10, 210), et les secondes extrémités (11, 211) des corps de connecteurs peuvent être raccordées l'une à l'autre.

12. Kit comprenant un connecteur anastomotique selon l'une quelconque des revendications précédentes et un manchon d'insertion (380) pouvant être engagé autour de la première extrémité (301) du corps de connecteur (301) pour recouvrir les dents (313), le manchon d'insertion (380) pouvant être retiré du corps de connecteur (301).

13. Kit selon la revendication 12, dans lequel le manchon d'insertion (380) est effilé à partir d'une première extrémité (382) pourvue d'une large ouverture jusqu'à une seconde extrémité (381) pourvue d'une ouverture étroite, dans lequel le corps de connecteur (301) peut être inséré dans le manchon d'insertion (380) à travers l'ouverture large, et dans lequel le manchon d'insertion (380) est déformable, de telle sorte que le corps de connecteur (301) peut déformer l'ouverture étroite et passer à travers celle-ci.

14. Kit selon la revendication 13, dans lequel la section de l'ouverture étroite est plus petite que la plus petite section transversale du corps de connecteur (301), lorsque le manchon d'insertion (380) n'est pas déformé.

15. Kit selon l'une quelconque des revendications 12 à 14, comprenant en outre une poignée de manœuvre (390) pouvant être engagée à l'intérieur du corps de connecteur (301) afin de pousser le corps de connecteur (1) dans le premier vaisseau (V1) et/ou le manchon d'insertion (380).
